# NEUE EUROPÄISCHE PATENTSCHRIFT

(11) **EP 0 847 242 B2**
(45) Veröffentlichungstag und Bekanntmachung des Hinweises auf die Entscheidung über den Einspruch: **11.03.2009**
(45) Hinweis auf die Patenterteilung: 24.10.2001
(21) Anmeldenummer: 96930096.1
(22) Anmeldetag: 24.08.1996
(51) Int. Cl.: A23G 3/00, A23L 1/236

(54) **ZUCKERFREIE DRAGIERTE PRODUKTE**
SUGAR-FREE DRAGEE-LIKE PRODUCTS
PRODUITS EN DRAGEES SANS SUCRE

(30) Priorität: 02.09.1995 DE 19532396
(43) Veröffentlichungstag der Anmeldung: 17.06.1998
(60) Teilanmeldung: 00100696.4
(73) Patentinhaber: Südzucker Aktiengesellschaft Mannheim/Ochsenfurt, 68165 Mannheim (DE)
(72) Erfinder: RAPP, Knut, M., D-67591 Offstein (DE); WILLIBALD-ETTLE, Ingrid, D-76829 Landau (DE)
(74) Vertreter: Schrell, Andreas
(86) Internationale Anmeldenummer: PCT/EP1996/003740
(87) Internationale Veröffentlichungsnummer: WO 1997/008958

(56) Entgegenhaltungen:
- EP-A- 0 431 376
- EP-A- 0 625 311
- EP-A- 0 625 578
- EP-A1- 0 625 578
- WO-A-89/07895
- WO-A-92/13866
- WO-A-95/07622
- DE-A- 3 715 919
- DE-A- 19 523 008
- DE-B2- 2 520 173
- US-A- 4 317 838
- US-A- 4 792 453
- US-A- 4 961 935
- US-A- 5 525 360
- DATABASE WPI Week 8748 Derwent Publications Ltd., London, GB; AN 87-337070 XP002022953 & JP,A,62 148 496 (MITSUI SEITO) , 2.Juli 1987
- DATABASE WPI Week 8748 Derwent Publications Ltd., London, GB; AN 87-337070 XP002022954 & JP,A,62 148 496 (MITSUI SEITO) , 2.Juli 1987
- SCHIWECK I. ET AL: 'New Developments in the Use of Sucrose as an Industrial Bulk Chemical' ZUCKERIND. Bd. 115, Nr. 7, 1990, Seiten 555 - 566
- SCHIWECK H.: 'Palatinit - Herstellung, technologische Eigenschaften und Analytik paltinithaltiger Lebensmittel' ALIMENTA Bd. 19, 1980, Seiten 5 - 16
- BOLLINGER H.: 'Palatinit (Isomalt) - ein kalorienreduzierter Zuckeraustauschstoff' GORDIAN Bd. 5, 1987, Seiten 92 - 95

## Beschreibung

Die vorliegende Erfindung betrifft verbesserte zukkerfreie Produkte.

Dragierte Produkte enthalten eine aus Zucker, Zukkeralkoholen, Schokoladearten und/oder anderen Glasuren hergestellte Decke und einen flüssigen, weichen oder festen Kern. Als Kerne werden zum Beispiel Kaugummi-Einlagen, Früchte, Komprimate oder auch pharmazeutische Produkte verwendet. So beschreibt US 4,792,453 einen zuckerfreien beschichteten Kaugummi, dessen Decke hydrierte Isomaltulose enthält. Dieses Kaugummi wird durch Dragieren mit einem Sirup gewonnen, der hydrierte Isomaltulose enthält. In dem Dragiersirup liegt somit 1-O-α-D-Glucopyranosyl-D-mannit (1,1-GPM) und 6-O-α-D-Glucopyranosyl-D-sorbit (1,6-GPS) gelöst in etwa äquimolaren Mengen vor.

Die bislang im Stand der Technik bekannten nichtdragierten Produkte sind verbesserungsfähig hinsichtlich ihrer Lagerfähigkeit, Süßkraft oder Löslichkeit. Diese Nachteile beruhen auf Art und Zusammensetzung der für die Herstellung der Produkte verwendeten Saccharide beziehungweie deren Gemische wie zum Beispiel hydrierte Isomaltulose. Hydrierte Isomaltulose entsteht durch Hydrierung von Isomaltulose und enthält die Komponenten 6-O-α-D-Glucopyranosyl-D-sorbit (im folgenden 1,6-GPS genannt) und 1-O-α-D-Glucopyranosyl-D-mannit (im folgenden 1,1-GPM genannt) in einem Verhaltnis von annähernd 1 zu 1. Hydrierte Isomaltulose löst sich nur mäßig in Wasser und neigt in gelöster Form bei Auftrag auf zu dragierende Oberflächen zum Verkleben.

Das der vorliegenden Erfindung zugrundeliegende technische Problem liegt somit darin, die vorstehenden Nachteile überwindende Produkte bereitzustellen.

Die Lösung dieses technischen Problems liegt in der Bereitstellung der in den Patentansprüchen gekennzeichneten Produkte.

Die Erfindung setzt 1,6-GPS angereicherte Gemische aus 1,6-GPS und 1,1-GPM in einem Verhältnis von größer 57 Gew.-% : kleiner 43 Gew.-% bis 99 Gew.-% : 1 Gew.-% (bezogen auf die Trockensubstanz des zur Herstellung eingesetzten Gemisches aus 1,6-GPS und 1,1-GPM, wobei dessen 1,6-GPS/1,1-GPM Gehalt gleich 100% ist) sowie 1,1-GPM angereicherte Gemische aus 1,6-GPS und 1,1-GPM in einem Verhältnis von 1 Gew.-% : 99 Gew.-% bis kleiner 43 Gew.-% : großer 57 Gew.-%, (bezogen auf die Trockensubstanz des zur Herstellung eingesetzten Gemisches aus 1,6-GPS und 1,1-GPM, wobei dessen 1,6-GPS/1,1,GPM Gehalt gleich 100% ist) ein. Für die Bereitstellung der erfindungsgemäßen Kaugummistreifen und -kissen können die Gemische je nach Zusammensetzung der für deren Herstellung verwendeten Ausgangssubstanz auch geringe Mengen an Sorbit, Mannit etc. enthalten. Die Gemische können erfindungsgemäß Zusatzstoff, wesentlicher Bestandteil oder im wesentlichen alleiniger Bestandteil verschiedenster Produkte im Lebensmittel- oder Arzneimittelbereich sein. Die erfindungsgemaß eingesetzten 1,6-GPS und 1,1-GPM angereicherten Gemische lassen sich besonders vorteilhaft aus einer einzigen Grundsubstanz, nämlich hydrierter Isomaltulose herstellen. Aus dieser kommerziell erhältlichen Grundsubstanz lassen sich also zwei Gemische mit jeweils unterschiedlichen Eigenschaften herstellen. Das 1,6-GPS angereicherte Gemisch zeichnet sich gegenüber hydrierter Isomaltulose und dem 1,1-GPM angereicherten Gemisch durch eine erhöhte Loslichkeit und größere Süßkraft aus. Die größere Süßkraft beruht einerseits darauf, daß 1,6-GPS schneller in Lösung geht und damit ein schnelles Süßempfinden auslöst und andererseits auf der objektiv größeren, der Verbindung 1,6-GPS eigenen Süßkraft. Das 1,1-GPM angereicherte Gemisch weist geringere Löslichkeit als hydrierte Isomaltulose auf. Die gezielte Verwendung dieser beiden Gemische in Produkten im Lebensmittel-, Süßmittel- oder Arzneimittelbereich ermöglicht es, den Produkten eine verbesserte Haltbarkeit und größere Süßkraft zu verleihen sowie deren Herstellungsverfahren zu vereinfachen.

Auch im Arzneimittelbereich spielt die Löslichkeit von Produkten vielfach eine bedeutende Rolle. Die Löslichkeit der Produkte beeinflußt unmittelbar die Wirkstoff-Freisetzung und damit auch den Wirkort und die Wirkzeit der applizierten Pharmazeutika.

Die erfindungsgemäßen Gemische können Farbstoffe, insbesondere Titandioxid enthalten.

In einer weiteren Ausfuhrungsform der Erfindung enthalten die in den erfindungsgemäßen Kaugummistreifen und -kissen eingesetzten Gemische zusatzlich ein oder mehrere Zuckeraustauschstoffe, insbesondere Xylit, Mannit, Sorbit, Maltit, Lactit oder Erythrit. Erfindungsgemäß ist es auch vorgesehen, daß die Gemische zusätzlich Fullstoffe, insbesondere Polydextrose, Calciumcarbonat oder Inulin enthalten können.

Die vorliegende Erfindung verwendet auch Gemische, die oberflächenaktive Substanzen wie Polysorbate (ethoxylierte Sorbitanester), insbesondere in einer Menge von 0,05 Gew.-% bis 0,5 Gew.-% und/oder Filmbildner wie Methylcellulose-Gelatine, Hydroxypropyl-Cellulose, Ethyl-Cellulose, HydroxyethylCellulose, Carboxymethyl-Cellulose und Gemische davon enthalten. Zusätzlich können Bindemittel wie Alginate, Pflanzengummis oder Weichmacher vorhanden sein.

In einer weiteren Ausführungsform verwendet die Erfindung Gemische, die Intensivsüßstoffe, insbesondere Cyclamat, Saccharin, Aspartam, Glycyrrhizin, Dihydrochalcon, Taumatin, Monellin, Acesulfam, Alitam oder Sucralose enthalten.

Die erfindungsgemäßen Produkte können zusätzlich Gelatine, Fett oder Fettersatzstoffe enthalten. Die erfindungsgemäßen Kaugummistreifen oder -kissen können selbstverständlich auch die bereits genannten Fullstoffe, Bindemittel, Farbstoffe, Intensivsüßstoffe, Emulgatoren, oberflächenaktive Substanzen, Zuckeraustauschstoffe, weitere Süßungsmittel oder pharmazeutische Wirkstoffe enthalten.

Die erfindungsgemäßen Hartkaramellen und Komprimate können selbstverständlich auch die bereits genannten Füllstoffe, Bindemittel, Farbstoffe, Intensivsüßstoffe, Emulgatoren, oberflächenaktive Substanzen, weitere Süßungsmittel oder pharmazeutische Wirkstoffe enthalten.

Die Erfindung betrifft Produkte, die als Hartkaramelle, Kaugummi-Kissen oder Kaugummi-Streifen ausgeführt sind.

Die erfindungsgemäß verwendeten Gemische können in vorteilhafter Weise in der Pharmazie eingesetzt werden.

Die Erfindung betrifft insbesondere ein Produkt, das als Komprimat ausgeführt ist. Erfindungsgemäße Komprimate können beispielsweise die zusammengepreßte, feste 1,1-GPM angereicherte Phase des erfindungsgemaß eingesetzten Gemisches enthalten. Bevorzugt ist auch ein Komprimat, das das erfindungsgemäß eingesetzte, zusammengepreßte, durch Eindampfen aus der flüssigen, 1,6-GPS angereicherten Phase erhaltene 1,6-GPS angereicherte Gemisch enthält. Die Komprimate eignen sich besonders zum Einschluß von Arzneimitteln und deren Applikation. Sie können beispielsweise in Form von Lutsch- oder Kautabletten ausgeführt sein.

Die Figuren zeigen:
- Figur 1: stellt die Zusammensetzung der 1,6-GPS und 1,1-GPM angereicherten Phasen dar, die aus einer auf 70°C erwarmten Suspension mit einem Trockensubstanzanteil von 75 Gew.% erhalten werden.
- Figur 2: stellt die Zusammensetzung der 1,6-GPS und 1,1-GPM angereicherten Phasen dar, die aus einer auf 70°C erwärmten Suspension mit einem Trockensubstanzanteil von 80 Gew.% erhalten werden.
- Figur 3: stellt die Zusammensetzung der 1,6-GPS und 1,1-GPM angereicherten Phasen dar, die aus einer auf 60°C erwärmten Suspension mit einem Trockensubstanzanteil von 75 Gew.% erhalten werden.
- Figur 4: stellt die Zusammensetzung der 1,6-GPS und 1,1-GPM angereicherten Phasen dar, die aus einer auf 60°C erwarmten Suspension mit einem Trockensubstanzanteil von 65 Gew.% erhalten werden.
- Figur 5: stellt die Zusammensetzung der 1,6-GPS und 1,1-GPM angereicherten Phasen dar, die aus einer auf 50 °C erwärmten Suspension mit einem Trockensubstanzanteil von 70 Gew.% erhalten werden.

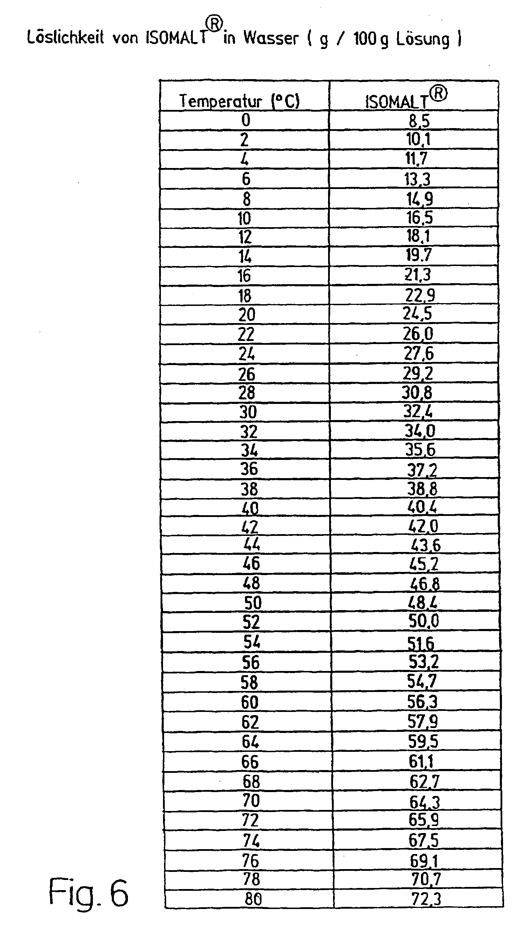

- Figur 6: stellt die Loslichkeit von hydrierter Isomaltulose (ISOMALT^{R}) in Wasser dar.
- Figur 7: stellt den Zusammenhang zwischen dem Trockensubstanzgehalt (Bx-Wert) mit hydrierter Isomaltulose (=ISOMALT^{R}) gesättigter Lösung und der Anfangskonzentration von hydrierter Isomaltulose in Wasser bei verschiedenen Temperaturen dar.
- Figur 8: stellt den Zusammenhang zwischen dem Verhältnis 1,6-GPS und 1,1-GPM in mit hydrierter Isomaltulose gesättigter Lösung und der Anfangskonzentration von hydrierter Isomaltulose in Wasser bei verschiedenen Temperaturen dar.
- Figur 9: stellt Auflösekinetiken von Komprimaten aus 1,6-GPS und 1,1-GPM dar.
- Figur 10: stellt die Abhängigkeit der Gewichtsänderung von Hartkaramellen von deren Zusammensetzung an 1,6-GPS und 1,1-GPM dar.

### Herstellbeispiel

### Herstellung 1,1-GPM und 1,6-GPS angereicherter 1,1-GPM/1,6-GPS Gemische bei 70°C (mit Gummi arabicum-Zusatz)

1920 g ISOMALT^{R} Typ M (hydrierte Isomaltulose) und 67,5 g Gummi arabicum (schnell-loslich) werden bei 80°C in 670,8 g Wasser gelöst und anschließend auf 70°C abgekuhlt. Unter Rühren werden dazu 341,7 g ISOMALT^{R} PF (Pulver) zugegeben. Der Wassergehalt von 3,5 Gew.-% in ISOMALT^{R} wurde dabei berücksichtigt.

Nach 60 min wird die feste von der flüssigen Phase getrennt. Dies kann beispielsweise durch Zentrifugation oder Filtration geschehen.

Im vorliegenden Beispiel wurde nach 20, 60, 120 und 180 min die Trennung der festen von der flüssigen Phase durch Filtration über eine auf 70° temperierte Drucknutsche vorgenommen. Die Zusammensetzungen der erhaltenen Phasen sind in folgender Tabelle I dargestellt:

**Tabelle I**

| Probe | 1 | 2 | 3 | 4 |
|---|---|---|---|---|
| Filtrat in g | 85,85 | 100,89 | 130,83 | 100,9 |
| Filterkuchen (feucht) | 37,76 | 51,55 | 54,76 | 32,34 |
| Filterkuchen (trocken) | 34,30 | 43,56 | 52,10 | 29,53 |
| Filtrat | 75,1:24.9 | 76,7:23,3 | 77:23 | 74,5:25,5 |
| (1,6-GPS : 1,1-GPM) % | | | | |
| Feststoff | 61:39 | 66,5:33,5 | 67,6:32,4 | 67,2:32,8 |
| (1,1-GPM : 1,6-GPS) % | | | | |

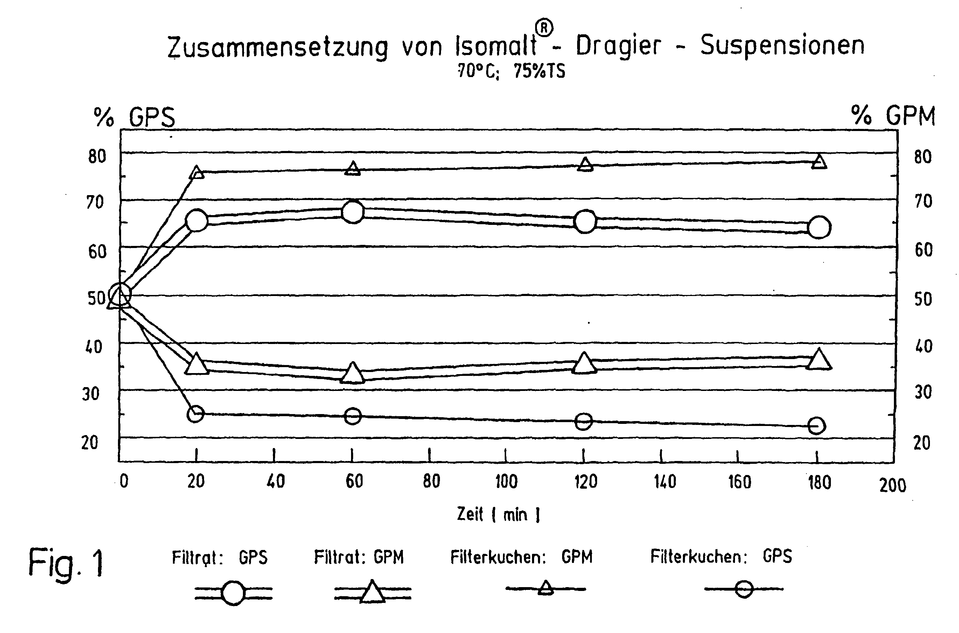

Die Ergebnisse sind in graphischer Form in Figur 1 dargestellt.

Nach 60 min ist in der flüssigen Phase 1,6-GPS auf ca. 75% angereichert, während in der festen Phase 1,1-GPM (ohne Kristallwasser berechnet) zu über 65% angereichert ist.

Die flüssige Phase kann durch Eindampfen oder Herabsetzen der Temperatur in Suspensionsform beziehungsweise in die feste Phase überführt werden.

Durch mehrmalige Wiederholung dieses Suspendierungs-Trennverfahrens mit den jeweils erhaltenen Phasen wird 1,6-GPS beziehungweise 1,1-GPM in reiner Form erhalten. Durch die Wahl geeigneter Temperaturen und Konzentrationen an hydrierter Isomaltulose sowie gegebenenfalls mehrmaliger Wiederholung des Trennverfahrens ist es erfindungsgemäß auch möglich, 1,1-GPM/1,6-GPS angereicherte Gemische gewünschter Zusammensetzung zu erhalten.

### Herstellung 1,1-GPM und 1,6-GPS angereicherter 1,1-GPM/1,6-GPS Gemische bei 35°C

5 kg Isomalt^{R} werden zu 5 kg Wasser (vollentsalzt) gegeben. Die Suspension wird bei 35 °C je nach Korngröße 1 - 20 Stunden gerührt.

Anschließend wird diese Suspension bei 35°C in flüssige Phase und feste Phase in einer beheizten Drucknutsche getrennt.

Die klare Losung wird im Rotationsverdampfer eingedampft und getrocknet, gegebenenfalls anschließend gemahlen.

Man erhält 1,95 kg weißen Feststoff (Wassergehalt vor Trocknen 24,8%, Verhältnis 1,1-GPM : 1,6-GPS 84% : 16%) und 7,86 kg klare Lösung (42,3°Brix, Verhältnis 1,1-GPM : 1,6-GPS 33,5% : 66,5%).

Die Trennung der beiden Phasen kann auch mittels einer Saugnutsche, Zentrifuge oder durch Sedimentation erfolgen.

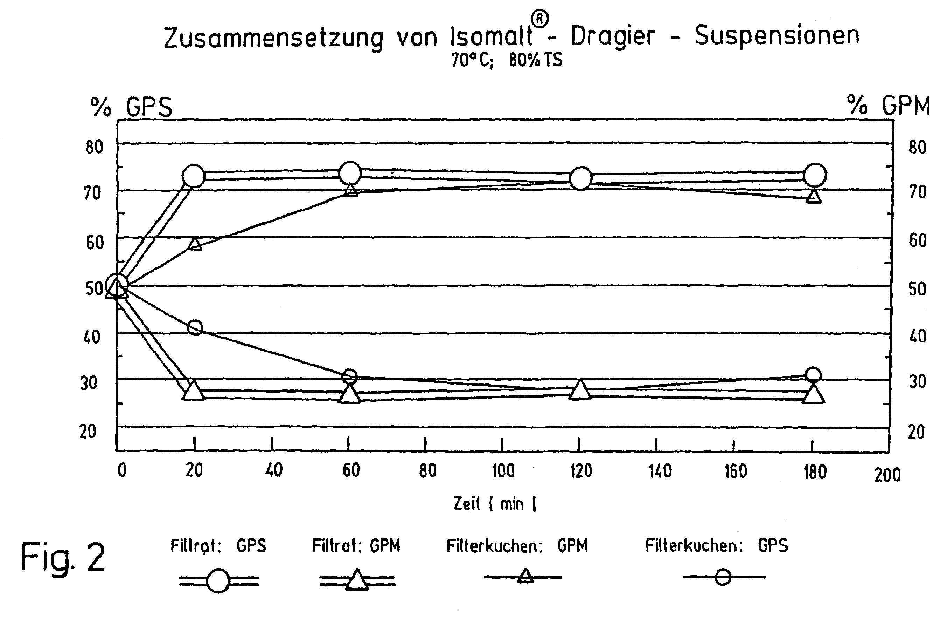

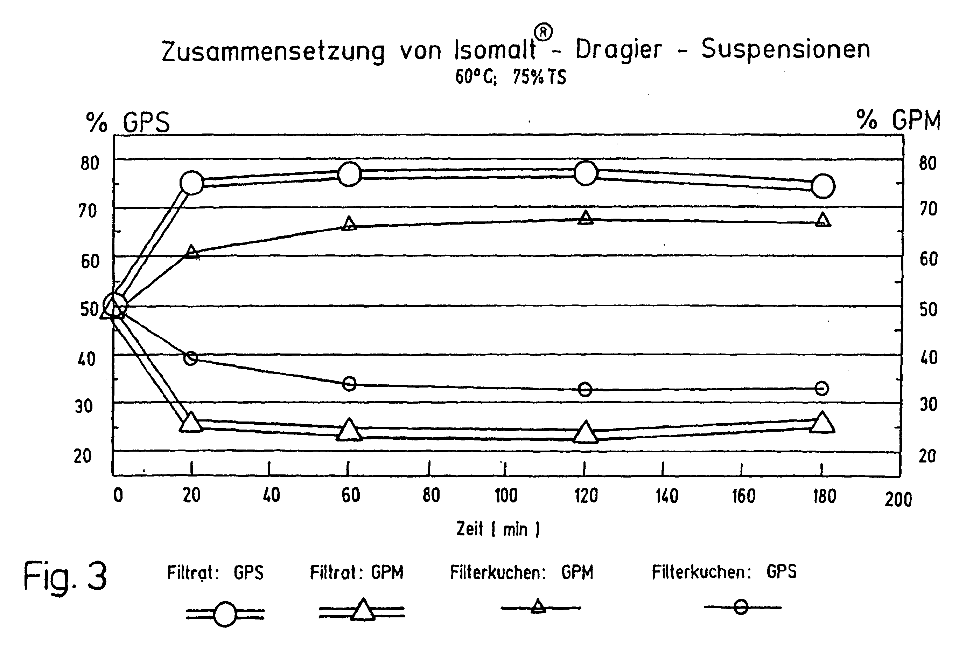

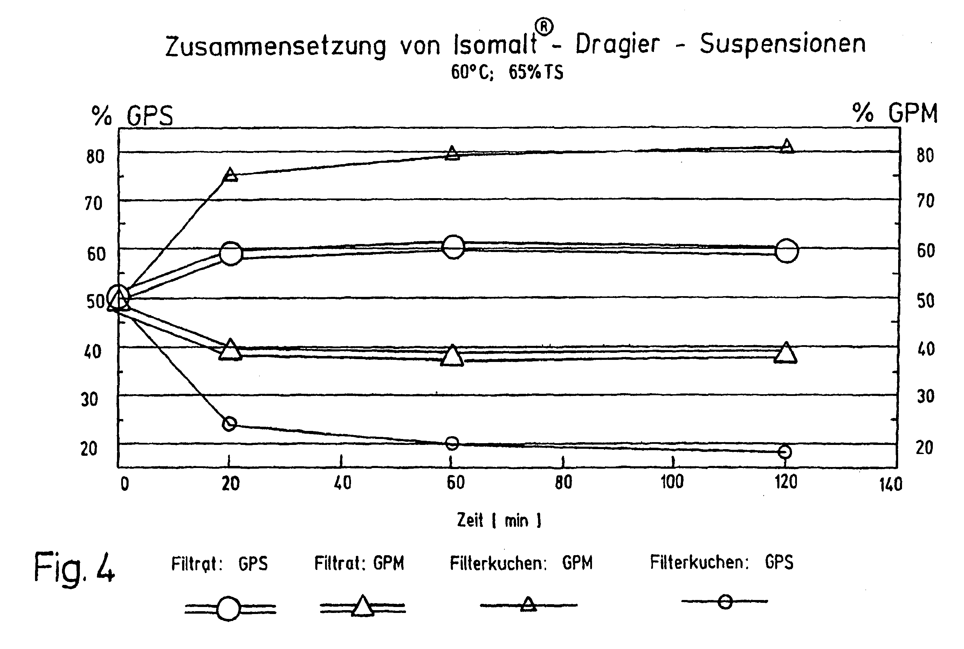

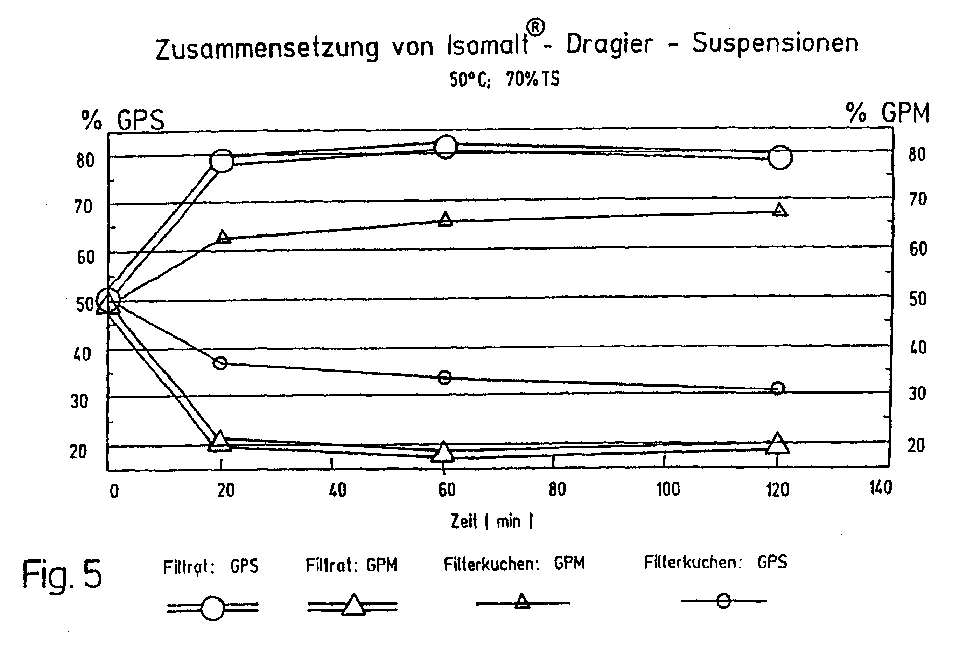

Das Herstellungsbeispiel sowie die Figuren 1 bis 5 verdeutlichen, daß durch gezielten Einsatz der Reaktionsparameter Temperatur und Feststoffkonzentration Gemische gewünschter Zusammensetzung erhalten werden können.

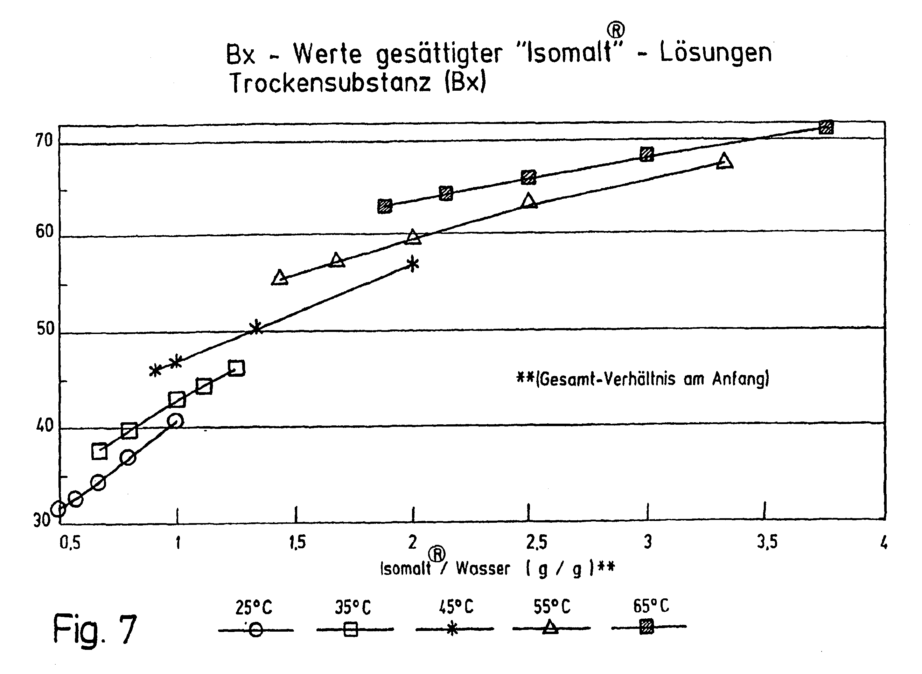

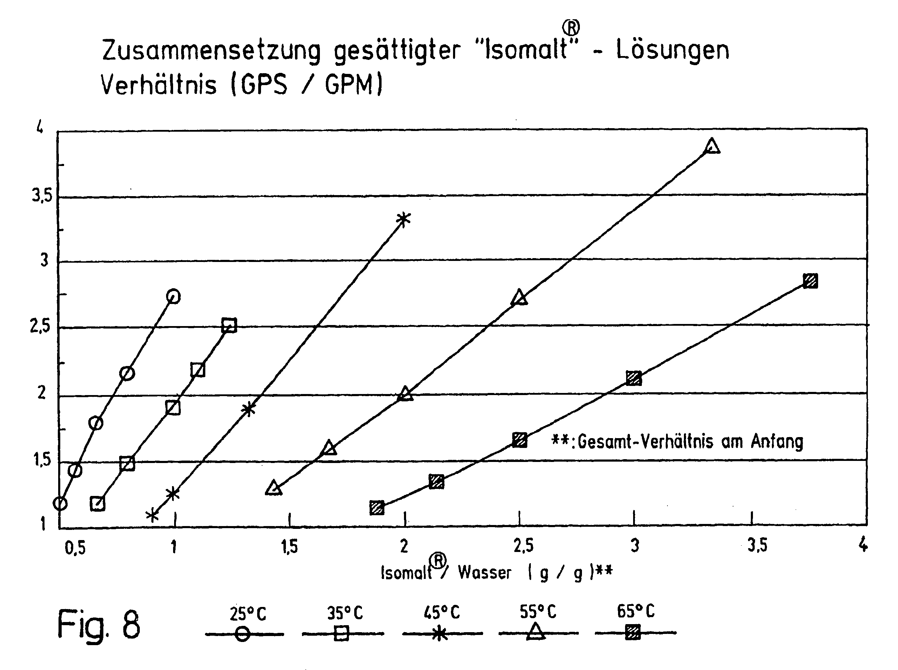

Die Figuren 7 und 8 illustrieren diesen erfindungsgemäßen Vorteil.

Diesen Figuren kann entnommen werden, in welchem Verhältnis hydrierte Isomaltulose (ISOMALT^{R}) mit Wasser gemischt und bei welcher Temperatur diese Suspension gehalten werden muß, um zum Beispiel eine flüssige Phase mit einem bestimmten 1,6-GPS : 1,1-GPM-Verhatnis zu erhalten.

Mischt man zum Beispiel ISOMALT^{R} mit Wasser im Verhältnis von 2:1, so erhält man bei einer Temperatur von 45°C eine ca. 57°-Brix-Lösung mit einem 1,6-GPS : 1,1-GPM-Verhaltnis von 77% : 23%, das heißt, 3,3 : 1.

Dasselbe Gemisch führt aber bei 55°C zu einer ca. 59°Brix-Lösung mit einem 1,6-GPS: 1,1-GPM-Verhältnis von 67% : 33%, das heißt, 2 : 1.

### Testbeispiel: Sensorische Analyse der Süßkraft von 1,1-GPM- beziehungsweise 1,6-GPS angereicherten 1,1-GPM/1,6-GPS-Mischungen

Für die Analyse der Süßkraft wurde als 1,1-GPM angereichertes Gemisch ein Gemisch mit einem Verhältnis von 1,1-GPM /1,6-GPS von 6,79 : 1 verwendet. Als 1,6-GPS angereichertes Gemisch wurde ein Gemisch mit einem Verhältnis von 1,6-GPS/1,1-GPM von 4,51 : 1 verwendet.

Die Gemische wurden den Probanden in Form wassriger Lösungen verabreicht.

### Schwellenwertermittlung:

**Tabelle II**

| Konzentrationsreine 1 (1,1-GPM) der Schwellenprü- fung | | |
|---|---|---|
| Probenkennzeichnung | Konzentration [g/100g] | Erkennung des Schwellenwertes [%] |
| 220895/1 | 0 | 0 |
| 220895/2 | 2 | 57,14 |
| 220895/3 | 4 | 14,28 |
| 220895/3 | 5 | 28,57 |
| 220895/4 | 6 | 0 |

**Tabelle III**

| Konzentrationsreihe 2 (1,6-GPS) der Schwellenprüfung | | |
|---|---|---|
| Probenkennzeichnung | Konzentration [g/100g] | Erkennung des Schwellenwertes [%] |
| 220895/5 | 0 | 0 |
| 220895/5 | 2 | 71,43 |
| 220895/7 | 4 | 14,28 |
| 220895/A | 5 | 14,28 |
| 220895/8 | 6 | 0 |

Bei Verwendung eines 1,6-GPS angereicherten Gemisches wird eine geringe Zuckeralkoholkonzentration eher erkannt als bei Verwendung eines 1,1-GPM angereicherten Gemisches.

### Unterschiedsprüfung:

Bei der paarweisen Unterschiedsprüfung (Duo-Test) zeigte sich, daß 62,5% (5 von 8 Probanden) die 1,6 GPS-reiche Mischung in einer 10 %iger Konzentration als süßer empfanden und 37,5% (3 von 8 Probanden) die 1,1-GPM-reiche Mischung.

### Beispiel 1:

### Herstellung von Kaugummi (Streifen) enthaltend ein 1,6-GPS angereichertes und ein 1,1-GPM angereichertes 1,1-GPM/1,6-GPS Gemisch

| Rezeptur | |
|---|---|
| Kaubase Nostic TWA | 1,5 kg |
| 1,6-GPS angereichertes Gemisch (76,5% 1,6-GPS, 23,5% 1,1-GPM) | 2,0 kg |
| Sorbitsirup (70% TS) | 0,6 kg |
| 1,1-GPM angereichertes Gemisch (85% 1,1-GPM, 15% 1,6-GPS) | 0,5 kg |
| Glycerin | 0,15 kg |
| Menthol | 0,15 kg |
| Aroma (Spearmint) | 0,1 kg |
| Aspartam | 2,5 g |
| Acesulfam K | 2,5 g |

### Herstellung

Die Kaugummibase wird in einem Warmeschrank bei ca. 55°C erwärmt, bevor sie in den Kneter gegeben wird, anschließend wird die Kaugummibase 1 - 2 Minuten geknetet. Während des Knetens werden die pulvrigen Zutaten (1,1-GPM und 1,6-GPS angereichertes Gemisch, Süßstoff, Menthol) nach und nach in der angegebenen Reihenfolge zugegeben, danach Aroma, Sorbitsirup und Glycerin. Geknetet wird, bis die Masse homogen ist (Endtemperatur ca. 45°C): Die Masse wird aus dem Kneter genommen und in ca. 1-kg-schwere Portionen geteilt.

Die portionierte Kaugummimasse wird ca. 15 - 20 Minuten auf einer Talkum bestreuten Unterlage zwischengelagert, mit einem geeigneten Extruder extrudiert und wie üblich weiterverarbeitet.

Der weitgehende Ersatz des leichtloslichen Zuckeralkohols Sorbit und der vollstandige Ersatz des ebenfalls leichtlöslichen Maltit durch die geringer löslichen Zuckeralkohole 1,6-GPS und 1,1-GPM führt zu dem sogenannten "long-lasting" Effekt (Geschmacksverstarkung).

Das Produkt ist insbesondere auch für Diabetiker geeignet.

### Beispiel 2

Herstellung von Hartkaramellen enthaltend ein 1,1-GPM angereichertes 1,1-GPM/1,6-GPS Gemisch

| Rezeptur | |
|---|---|
| 1,1-GPM angereichertes Gemisch (85% 1,1-GPM, 15% 1,6-GPS) | 25 kg |
| Wasser | 8 kg |
| Zitronensaure | 0,3 kg |
| Aroma (Ananas) | 0,1 kg |
| Acesulfam K | 25 g |

### Herstellung

Das 1,1-GPM angereicherte Gemisch und Wasser werden im Bonbonkocher auf 155 - 160°C gekocht, 5 Minuten vollem Vakuum ausgesetzt und nach Abkühlen der Masse auf 110 - 115°C werden Säure, Aroma und Süßstoff zugegeben. Anschließend wird die Masse zu Bonbons geprägt und gekühlt.

Alternativ kann die obige Rezeptur ohne den Wasserzusatz direkt in einer Schmelzextrusion zu Bonbons verarbeitet werden. Das Schmelzextrusionsverfahren kann selbstverständlich auch angewendet werden, falls Hartkaramellen aus 1,6-GPS angereicherten Gemisch hergestellt werden.

1,1-GPM angereicherte Hartkaramellen bilden an der Oberfläche eine mikrokristalline Grenzschicht aus 1,1-GPM-Dihydrat aus, die zu einer verminderten Klebrigkeit führt und die weitere Wasseraufnahme aus der Atmosphäre reduziert (günstiges Lagerverhalten). Die Produkte sind diabetikergeeignet.

Desweiteren weisen 1,1-GPM angereicherte Hartkaramellen eine erhöhte Temperaturstabilität auf. Die Temperaturstabilität wird durch den Glasübergangspunkt Tg°C beschrieben, d.h. 1,1-GPM angereicherte Hartkaramellen haben einen höheren Glasübergangspunkt (Tg = 65,6°C +/- 1,8°C im Vergleich zu Isomalt^{R} Hartkaramellen Tg = 57,5°C +/- 1,7°C).

### Beispiel 3

### Herstellung eines Komprimates aus 1,1-GPM und 1,6-GPS angereicherten 1,1-GPM/1,6-GPS Gemischen

| Rezeptur | | | |
|---|---|---|---|
| | | Fruchtaroma | Mintaroma |
| a) | 1,1-GPM angereichertes Gemisch (85% 1,1-GPM, 15% 1,6-GPS) | 9,9 kg | 9,9 kg |
| b) | 1,6-GPS angereichertes Gemisch (83% 1,6-GPS, 17% 1,1-GPM) | 9,9 kg | 9,9 kg |
| Acesulfam K | | 15 g | 15 g |
| | Zitronensäure | 30 g | - |
| | Aroma | 50 g | 50 g |
| | Magnesiumstearat | 50 g | 50 g |

### Herstellung von Lutschtabletten (Kautabletten)

Die Komponenten werden gemischt und in einer Exzenterpresse unter folgenden Bedingungen gepreßt:
Preßkraft 20 - 70 kN
spezifische Preßkraft 0,2 - 0,9 kN/mm²

Für Lutschtabletten wird a) 1,1-GPM angereichertes Gemisch, für Kautabletten wird b) 1,6-GPS angereichertes Gemisch verwendet.

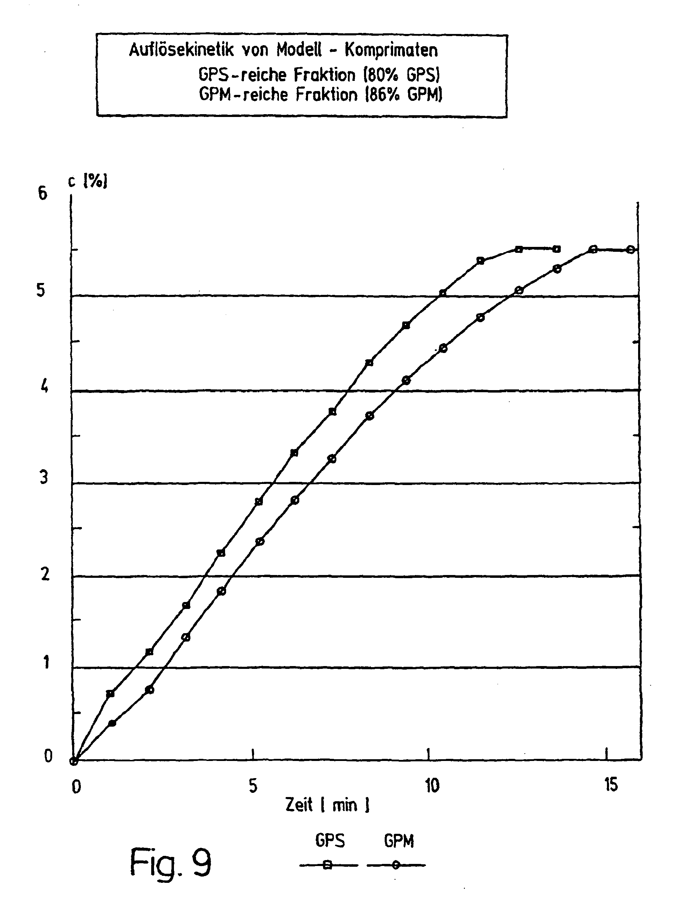

Aufgrund der geringen Löslichkeit des 1,1-GPM angereicherten Gemisches wird ein langsames Auflösen und damit ein verlängertes Freisetzen von Aroma oder Wirkstoffen bei pharmazeutischen Komprimaten bewirkt. Figur 9 illustriert die gegenüber einem 1,6-GPS angereicherten Komprimat geringere Löslichkeit eines 1,1-GPM angereicherten Komprimats. Die 1,6-GPS angereicherten Komprimate wurden ohne Hilfsmittel bei 70 kN, die 1,1-GPM angereicherten Komprimate bei 50 kN, ebenfalls ohne Hilfsmittel, verpreßt.

### Beispiel 4

Herstellung gefüllter Hartkaramellen, wobei die Hülle 1,1-GPM angereichertes Gemisch und die flüssige Füllung 1,6-GPS angereichertes Gemisch enthält

| 1. Hartkaramelmasse | |
|---|---|
| 1,1-GPM angereichertes Gemisch (85% 1,1-GPM, 15% 1,6-GPS) | 25 kg |
| Wasser | 8 kg |
| Zitronensäure | 0,3 kg |
| Zitronenaroma | 0,03 kg |

### Herstellung

Das 1,1-GPM angereicherte Gemisch und Wasser werden im Bonbonkocher bei 155 -160°C gekocht, 5 Minuten vollem Vakuum ausgesetzt und anschließend Säure und Aroma zugegeben. Die Schmelze wird auf 65 - 70°C im Kegelroller abgekuhlt.

### 2. Füllung

| Rezeptur | |
|---|---|
| Raftilose L95 (Fructooligosaccharide) | 2,5 kg |
| 1,6-GPS angereichertes Gemisch (82% 1,6-GPS,18% 1,1-GPM) | 5,9 kg |
| Wasser | 1,5 kg |
| Zitronensäure | 0,09 kg |
| Zitronenaroma | 0,01 kg |

### Herstellung

Raftilose L95 wird mit Wasser auf 80°C erwärmt, darin wird feinpulvriges 1,6-GPS angereichertes Gemisch aufgelost, nach Abkühlen auf 70°C wird Säure und Aroma zugegeben und als Füllung im Kegelroller in die plastische Schmelze aus 1,1-GPM angereichertem Gemisch verarbeitet. Die Füllung beträgt ca. 10 - 15% der Gesamtbonbonmasse.

Die Decke der gefüllten Hartkaramellen ist stabil gegen atmospharische Wasseraufnahme (gutes Lagerverhalten), die Füllung ist flüssig und wegen Fehlens von Maltitsirup diabetikergeeignet.

### Beispiel 5:

### Herstellung und Untersuchung erfindungsgemäßer Hartkaramellen

Zur Herstellung verschiedener Hartkaramellen wurden als Rohstoffe hydrierte Isomaltulose (1) (ISOMALT^{R}) und die erfindungsgemaß eingesetzten Gemische (2) bis (7) die keine weiteren Zuckeraustauschstoffe enthalten, wie in Tabelle IV ausgeführt, eingesetzt (TS: Trockensubstanz).

**Tabelle IV**

| Rohstoffbezeichnung | 1,1-GPM Anteil (% TS) | 1,6-GPS-Anteil (% TS) |
|---|---|---|
| Isomalt^{R} (1) | 48,6 | 50,3 |
| Isomalt PU-3,3/55-F (2) | 16,7 | 81,3 |
| Isomalt PU-1,9/45-F (3) | 23,1 | 75,1 |
| Isomalt PU-1/35-F (4) | 32,8 | 65,6 |
| Isomalt PU-3,3/55-FK (5) | 60,7 | 38,3 |
| Isomalt PU-1,9/45-FK (6) | 72,4 | 26,8 |
| Isomalt PU-1/35-FK (7) | 83,2 | 16,4 |

Die Karamellen wurden bei 70 % relativer Feuchte bei 25°C unterschiedliche Zeit gelagert (Wassergehalt der Karamellen: 1,5 %).

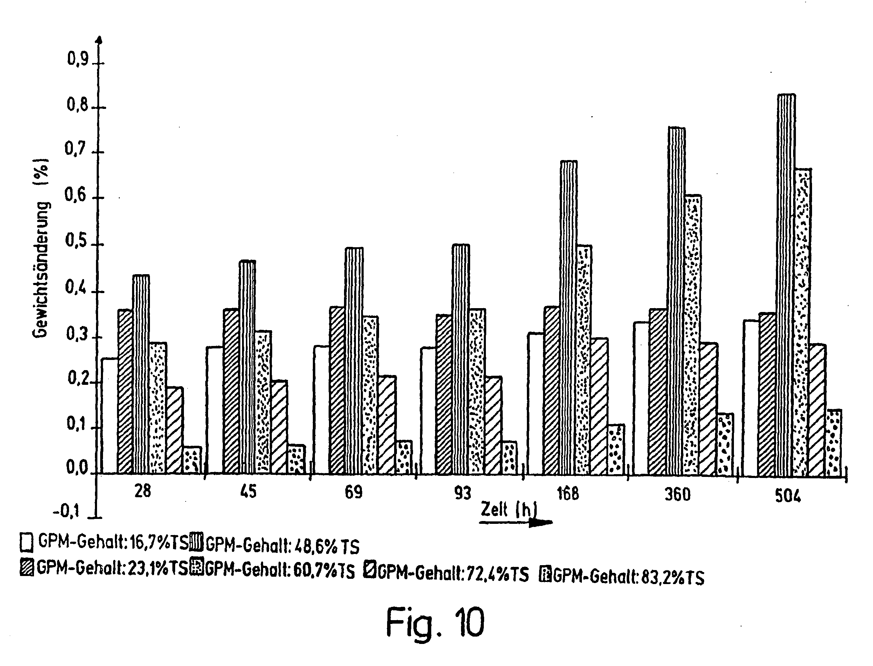

Die Fig. 10 verdeutlicht, daß die Hartkaramellen aus herkömmlicher hydrierter Isomaltulose im Vergleich zu den erfindungsgemäßen Produkten eine erheblich erhohte Wasseraufnahme im Lagertest aufweisen. Die erfindungsgemäßen Karamellen sind daher erheblich besser lagerfahig.

## Patentansprüche

1. Zuckerfreie Hartkaramelle, enthaltend als alleinige Zuckeraustauschstoffe ein Gemisch bestehend aus 1,6-GPS (6-O-α-D-Glucopyranosyl-D-sorbit) und 1,1-GPM (1-O-α-D-Glucopyranosyl-D-mannit) in einem Verhältnis von 1 Gew.-% : 99 Gew.-% bis kleiner 43 Gew.-% : größer 57 Gew.-%.

2. Zuckerfreier Kaugummistreifen oder Kaugummikissen, enthaltend mindestens einen Intensivsüßstoff sowie ein Gemisch bestehend aus 1,6-GPS 6-O-α-D-Glucopyranosyl-D-sorbit und 1,1-GPM 1-O-α-D-Glucopyranosyl-D-mannit in einem Verhältnis von größer 57 Gew.-% kleiner 43 Gew.-% bis 99 Gew.-% : 1 Gew.-%.

3. Zuckerfreies Komprimat, enthaltend als alleinige Zuckeraustauschstoffe ein Gemisch bestehend aus 1,6-GPS 6-O-α-D-Glucopyranosyl-D-sorbit und 1,1-GPM 1-O-α-D-Glucopyrasonyl-D-mannit in einem Verhältnis von größer 57 Gew.-% : kleiner 43 Gew.-% bis 99 Gew.-% : 1 Gew.-% oder bestehend aus 1,6-GPS 6-O-α-D-Glucopyranosyl-D-sorbit und 1,1-GPM 1-O-α-D-Glucopyranosyl-D-mannit in einem Verhältnis von 1 Gew.-% : 99 Gew.-% bis kleiner 43 Gew.-% : größer 57 Gew.-%.

## Claims

1. Sugar-free hard caramel containing as sole sugar substitutes a mixture consisting of 1,6-GPS (6-O-α-D-glucopyranosyl-D-sorbitol) and 1,1-GPM (1-O-α-D-glucopyranosyl-D-mannitol) in a ratio of 1 % w/w : 99 % w/w to less than 43 % w/w : more than 57 % w/w.

2. Sugar-free chewing gum strip or chewing gum dragee containing at least one intense sweetener as well as a mixture consisting of 1,6-GPS (6-O-α-D-glucopyranosyl-D-sorbitol) and 1,1-GPM (1-α-a-D-glucopyranosyl-D-mannitol) in a ratio of more than 57 % w/w : less than 43 % w/w to 99 % w/w : 1 % w/w.

3. Sugar-free compressed product containing as sole sugar substitutes a mixture consisting of 1,6-GPS (6-O-α-D-glucopyranosyl-D-sorbitol) and 1,1-GPM (1-O-α-D-glucopyranosyl-D-mannitol) in a ratio of more than 57 % w/w : less than 43 % w/w to 99 % w/w : 1 % w/w or consisting of 1,6-GPS (6-O-α-D-glucopyranosyl-D-sorbitol) and 1,1-GPM (1-O-α-D-glucopyranosyl-D-mannitol) in a ratio of 1 % w/w : 99 % w/w to less than 43 % w/w : to more than 57 w/w.

## Revendications

1. Caramel dur sans sucre contenant, en tant que succédané de sucre unique, un mélange composé de 1,6-GPS (6-O-α-D-glucopyranosyl-D-sorbitol) et 1,1-GPM (1-O-α-D-glucopyranosyl-D-mannitol) selon un rapport de 1 % en poids : 99 % en poids à moins de 43 % en poids : plus de 57 % en poids.

2. Chewing-gum sans sucre en forme de bande ou de coussinet contenant au moins un édulcorant intense ainsi qu'un mélange composé de 1,6-GPS (6-O-α-D-glucopyranosyl-D-sorbitol) et 1,1-GPM (1-O-α-D-glucopyranosyl-D-mannitol) selon un rapport de plus de 57 % en poids : moins de 43 % en poids à 99 % en poids : 1 % en poids.

3. Comprimé sans sucre contenant, en tant que succédané de sucre unique, un mélange composé de 1,6-GPS (6-O-α-D-glucopyranosyl-D-sorbitol) et 1,1-GPM (1-O-α-D-glucopyranosyl-D-mannitol) selon un rapport de plus de 57 % en poids : moins de 43 % en poids à 99 % en poids : 1 % en poids ou composé de 1,6-GPS (6-O-α-D-glucopyranosyl-D-sorbitol) et 1,1-GPM (1-O-α-D-glucopyranosyl-D-mannitol) selon un rapport de 1 % en poids : 99 % en poids à moins de 43 % en poids : plus de 57 % en poids.
